# FASCICULE DE BREVET EUROPEEN

(11) **EP 1 426 035 B1**
(45) Date de publication et mention de la délivrance du brevet: **14.03.2007**
(21) Numéro de dépôt: 03292500.0
(22) Date de dépôt: 09.10.2003
(51) Int. Cl.: A61Q 5/00, A61K 8/19, A61K 8/25, A61K 8/26, A61K 8/84

(54) **Composition cosmétique comprenant des particules minérales et une polyéthylèneimine**
Kosmetische Zusammensetzungen, die Mineralteilchen und ein Polyethyleneimin enthalten
Cosmetic composition comprising mineral particles and a polyethyleneimine

(30) Priorité: 02.12.2002 FR 0215175
(43) Date de publication de la demande: 09.06.2004
(73) Titulaire: L'ORÉAL, 75008 Paris (FR)
(72) Inventeur: Perron, Béatrice, 78350 Jouy en Josas (FR); Paul, Laurence, 95320 Saint Leu La Foret (FR); Restle, Serge, 95390 Saint-Prix (FR)
(74) Mandataire: Dodin, Catherine

(56) Documents cités:
- DE-A- 19 752 837
- FR-A- 2 822 687
- GB-A- 2 367 749
- US-A- 3 819 827

## Description

La présente invention est relative à une composition cosmétique, notamment capillaire, contenant, dans un milieu cosmétiquement acceptable, des particules minérales et au moins une polyalkylèneimine. Elle vise également un procédé de traitement cosmétique des cheveux comprenant l'application de cette composition ainsi que son utilisation notamment en tant que produit capillaire rincé.

Pour le nettoyage et/ou le lavage des cheveux, l'utilisation de compositions capillaires détergentes (ou shampooings), à base essentiellement d'agents tensioactifs classiques de types notamment anionique, non-ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés de silicone, qui confèrent en effet aux cheveux lavés, secs ou mouillés, une facilité de démêlage et une douceur nettement accrue par rapport à ce qui peut être obtenu avec les compositions nettoyantes correspondantes qui en sont exemptes.

Cependant, ces avantages cosmétiques s'accompagnent malheureusement également, sur cheveux séchés, de certains effets cosmétiques jugés indésirables, à savoir un alourdissement de la coiffure, un manque de lissage.

En outre, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion entre les fibres affectant le coiffage. Ces inconvénients sont accentués dans le cas de cheveux fins, qui manquent de nervosité et de volume.

Il a déjà été proposé l'utilisation de particules dans des compositions rincées, de façon à améliorer le toucher et l'aspect des cheveux. A titre d'illustration, le brevet US 5 334 376 propose l'ajout de particules de carbonate de calcium dans des compositions de conditionnement des cheveux contenant une silicone, un alcool gras et une amide.

Dans la demande de brevet DE 199 46 784, il a également été proposé l'utilisation de particules de différents oxydes, hydroxydes, carbonates, silicates ou phosphates, dans des compositions capillaires, pour réduire l'aspect gras des cheveux. Il est prévu, en toutes généralités, d'associer ces particules aux ingrédients classiques des shampooings.

FR2822687 divulgue des compositions comprenant du sulfate de barium et un polymère cationique. DE19752837 décrit amélioration de la tenue de la coiffure par les polyéthylèneimines. Aucun de ces documents ne divulgue un rapport particulier entré les particules et les PEI.

Toutefois, et malgré les progrès réalisés récemment dans le domaine des produits capillaires rincés et notamment des shampooings, ces derniers ne donnent pas vraiment complètement satisfaction, de sorte qu'un fort besoin existe encore actuellement quant à pouvoir disposer de nouveaux produits présentant, au niveau de l'une ou de plusieurs de leurs propriétés, de meilleures performances.

La Demanderesse a découvert, de manière surprenante et inattendue, qu'en choisissant l'agent de conditionnement, associé avec des particules minérales particulières, il était possible d'améliorer les résultats obtenus avec les produits cosmétiques, notamment capillaires rincés, en terme de propriétés cosmétiques et de mise en forme. En particulier, on apporte de la texture aux cheveux (sensation d'épaisseur accrue) et une meilleure tenue de la coiffure.

L'invention a pour objet une composition cosmétique, notamment capillaire, comprenant, dans un milieu cosmétiquement acceptable :
(a) des particules solides minérales comprenant au moins un élément de la colonne IIa du tableau de classification périodique des éléments et, (b) au moins une polyalkylèneimine ,
le rapport pondéral polyalkylèneimine/ particules minérales allant de 0,1 à 0,0001.

Un autre objet de la présente invention consiste en un procédé de traitement cosmétique des cheveux mettant en oeuvre la composition selon l'invention.

L'invention a encore pour objet l'utilisation de la composition pour apporter de la texture aux cheveux (sensation d'épaisseur accrue) et une meilleure tenue de la coiffure.

L'invention a encore pour objet l'utilisation de la composition en cosmétique capillaire, notamment en application capillaire rincée, notamment comme shampooing.

D'autres objets, caractéristiques, aspects et avantages de l'invention apparaîtront encore plus clairement à la lecture de la description et des divers exemples qui suivent.

### (a) PARTICULES MINERALES

Les particules minérales solides comprennent au moins un élément de la colonne IIa du tableau de classification périodique des éléments. Elles sont notamment choisies parmi les particules contenant au moins 10 % en poids de carbonate de calcium ou d'au moins un silicate, l'oxyde de magnésium, le sulfate de baryum et leurs mélanges.

Les particules minérales solides présentent, de préférence, une taille primaire moyenne en nombre comprise entre 2 nm et 2 microns, plus préférentiellement entre 5nm et 500 nm, et plus préférentiellement encore entre 10 nm et 250 nm.

Les particules selon l'invention peuvent, par exemple, avoir une forme quelconque, par exemple la forme de sphères, de paillettes, d'aiguilles, de plaquettes ou des formes totalement aléatoires. De préférence, elles sont sensiblement sphériques.

Au sens de la présente invention, on entend par "*taille primaire de particule*", la dimension maximale qu'il est possible de mesurer entre deux points diamétralement opposés d'une particule individuelle. La taille peut être déterminée, par exemple, par microscopie électronique à transmission ou à partir de la mesure de la surface spécifique par la méthode BET ou bien par l'intermédiaire d'un granulomètre laser.

Conformément à la présente invention, la particule peut être une particule massique formée entièrement de carbonate de calcium. Le carbonate de calcium peut également constituer totalement ou partiellement le coeur de la particule, ce dernier étant recouvert d'un autre constituant, comme par exemple, un oxyde, un silicate ou un métal. Le carbonate de calcium peut encore former uniquement le revêtement d'un substrat de constitution chimique différente, comme par exemple un oxyde, un silicate ou un métal.

Dans le cas où les particules sont formées par du carbonate de calcium et d'autres charges, le carbonate de calcium se trouve à l'état libre et ne forme pas de liaisons chimiques avec les autres charges. Il s'agit alors d'un alliage entre le carbonate de calcium et d'autres charges, notamment avec des oxydes de métaux ou de métalloïdes, en particulier obtenu par fusion thermique de ces différents constituants.

Lorsque les particules contenant au moins 10 % en poids de carbonate de calcium comprennent, en outre, un oxyde de métal ou de métalloïde, celui-ci est notamment choisi parmi l'oxyde de silicium, de bore ou d'aluminium.

De préférence, les particules contiennent au moins 50 % en poids de carbonate de calcium, mieux encore au moins 70 % en poids, et les particules constituées à plus de 90 % en poids de carbonate de calcium sont particulièrement préférées selon la présente invention.

Plus avantageusement encore, les particules contenant au moins 10 % en poids de carbonate de calcium sont des particules de carbonate de calcium substantiellement pur.
Le carbonate de calcium convenant dans les compositions de la présente invention peut être d'origine naturelle ou peut être d'origine synthétique. Dans ce dernier cas, il peut être obtenu à partir d'oxyde de calcium, de peroxyde de calcium, d'acétate ou d'éthylate de calcium.

Le silicate utilisable selon l'invention peut être le silicate de magnésium. On peut notamment utiliser les composés commercialisés par la société LAPORTE sous la dénomination LAPONITE XLG et LAPONITE XLS.

Lorsque les particules contenant au moins 10% en poids d'au moins un silicate comprennent en outre un oxyde de métal ou de métalloïde, celui-ci est notamment choisi parmi l'oxyde de silicium, de bore ou d'aluminium.

Les silicates convenant dans les compositions de la présente invention peuvent être d'origine naturelle ou d'origine synthétique.

Les particules minérales selon l'invention sont, notamment, utilisées en une quantité comprise entre 0,01 % et 30 % en poids, et de préférence entre 0,05 % et 10 % en poids, et plus particulièrement entre 0,1 % et 5 % en poids par rapport au poids total de la composition.

La composition selon l'invention peut également contenir d'autres types de particules, par exemple, des particules d'oxyde de titane ou de zinc.

### (b)POLYALKYLENEIMINE

Les polyalkylèneimines utilisées préférentiellement selon l'invention sont des polymères contenant de 6 à 20.000 motifs de répétition. De préférence, on choisit des polyalkylène imines comprenant au moins 5% d'amines tertiaires, avantageusement au moins 10% de fonctions amines tertiaires, et encore plus préférentiellement au moins 20%. Ces polymères peuvent être des homopolymères ou des copolymères, linéaires, branchés ou de structures dendrimériques.

Ces polymères comprennent les motifs répétitifs suivants : 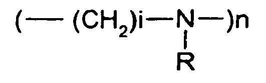 dans lesquels :
i représente un entier supérieur ou égal à 2 et de préférence inférieur ou égal à 6, préférentiellement i=2 ;
n représente un entier variant de 6 à 20.000 et de préférence de 8 à 2500
R représente un atome d'hydrogène ou un motif 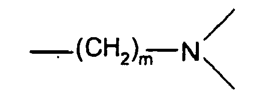
dans lequel m représente un entier supérieur ou égal à 2, préférentiellement m=2 ;

Ces polymères sont généralement terminés par des fonction amines terminales notamment des amines primaires.

Selon l'invention, les polyalkylèneimines sont de préférence des polyalkylène(C2-C4)imines et plus particulièrement des polyéthylèneimines.

Les polyalkylèneimines utilisées conformément à l'invention ont en général un poids moléculaire moyen en poids allant de 300 à 100.000 et de préférence de 350 à 50.000 et plus particulièrement de 400 à 10.000 et préférentiellement de 500 à 2000.
Les polyalkylèneimines ayant ces poids moléculaires permettent d'améliorer la dispersion des particules minérales et en conséquence leur dépôt.

Les poids moléculaires peuvent être déterminés par diffusion quasi élastique de la lumière.

Les polyalkylèneimines ont de préférence une densité de charge cationique inférieure ou égale à 20 meq/g et, de préférence, supérieure ou égale à 0,05meq/g., et plus particulièrement de 4 à 20 meq/g.

La densité de charge peut être déterminée selon la méthode Kjeldahl ou calculée.

Les polyalkylèneimines sont notamment décrites dans l'ouvrage "Polymer Science dictionary" 2^{ème} édition, Mark ALGER, CHAPMAN & HALL, 1997.

Les polyalkylèneimines sont présents, dans les compositions selon l'invention, dans des proportions allant, de préférence, de 0,0005 à 5 % en poids et de préférence de 0,001 à 1 % en poids par rapport au poids total de la composition et plus particulièrement de 0,005 à 0,5% en poids et encore plus préférentiellement de 0,005 à 0,25% en poids.

La ou les polyalkylèneimines peuvent être alors utilisées en un rapport pondéral avec les particules minérales allant de 0,1 à 0,0001, de préférence de 0,05 à 0,001.

Les compositions de l'invention comprennent de préférence en outre au moins un tensioactif qui est généralement présent en une quantité comprise entre 0,2% et 40% en poids environ, de préférence entre 1% et 35% et encore plus préférentiellement entre 1,5 % et 30%, par rapport au poids total de la composition.

Les tensioactifs convenant à la mise en oeuvre de la présente invention peuvent être de toute nature et de préférence sont solubles dans l'eau à température ambiante :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkyl sulfates, les alkyl éther sulfates, alkyl amido éther sulfates, alkyl aryl polyéther sulfates, monoglycérides sulfates ; les alkyl sulfonates, alkylphosphates, alkyl amido sulfonates, alkyl aryl sulfonates, α-oléfine-sulfonates, paraffine-sulfonates ; les alkyl sulfo succinates, les alkyl éther sulfosuccinates, les alkyl amide sulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyllactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkylamido(C₆-C₂₄) éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant 1 à 5 groupements glycérol et en particulier 1,5 à 4; les esters d'acides gras du sorbitan oxyéthylénés ayant de 2 à 30 moles d'oxyde d'éthylène ; les esters d'acides gras du sucrose, les esters d'acides gras de polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s) ou zwittérionique(s) :

Les agents tensioactifs amphotères ou zwittérioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits commercialisés sous la dénomination MIRANOL, tels que décrits dans les brevets US-2528378 et US-2781354 et classés dans le dictionnaire CTFA, 3ème édition, 1982, sous les dénominations Amphocarboxy-glycinates et Amphocarboxypropionates de structures respectives :

R₂-CONHCH₂CH₂-N(R₃)(R₄)(CH₂COO-) (2)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R_{2'}-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂- CHOH - SO₃H
R_{2'} désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

A titre d'exemple on peut citer le cocoamphodiacetate commercialisé sous la dénomination commerciale MIRANOL C2M concentré NP par la Société RHODIA CHIMIE.

### (iv) Tensioactifs cationiques :

Les tensioactifs cationiques peuvent être choisis parmi :
A) les sels d'ammonium quaternaires de la formule générale (XII) suivante : 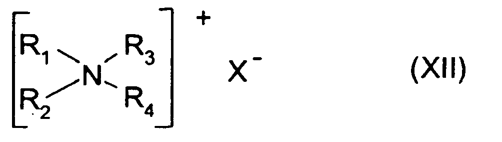 dans laquelle X⁻ est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate.
   , et
   i) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
      R4 désigne un radical alkyle, linéaire ou ramifié, comportant de 16 à 30 atomes de carbone.
      De préférence le tensioactif cationique est un sel (par exemple chlorure) de cétyl triméthyl ammonium.
   ii) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
      R3 et R4, identiques ou différents, désignent un radical alkyle, linéaire ou ramifié, comportant de 12 à 30 atomes de carbone, ledit radical comprenant au moins une fonction ester ou amide.
      R3 et R4 sont notamment choisis parmi les radicaux alkyl(C₁₂-C₂₂)amido alkyle(C₂-C₆), alkyl(C₁₂-C₂₂)acétate ;
      De préférence le tensioactif cationique est un sel (par exemple chlorure) de stéaramidopropyl diméthyl (myristylacétate) ammonium.
B) - les sels d'ammonium quaternaire de l'imidazolinium, comme par exemple celui de formule (XIII) suivante : 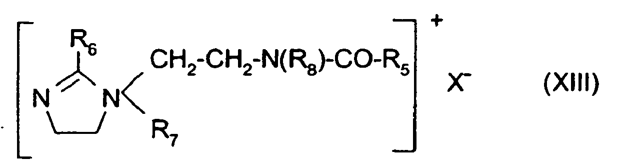 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
C) - les sels de diammonium quaternaire de formule (XIV) : 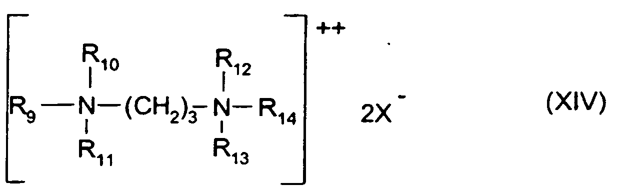 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
D) - les sels d'ammonium quaternaire contenant au moins une fonction ester de formule (XV) suivante : 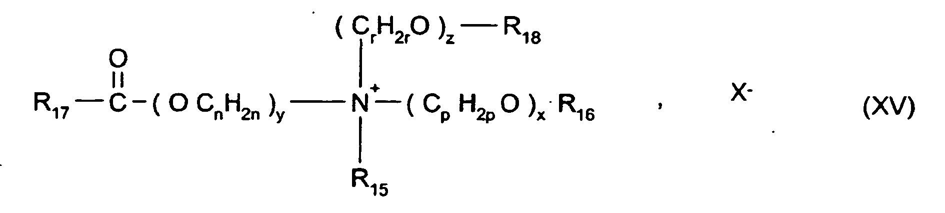 dans laquelle :
   - R₁₅ est choisi parmi les radicaux alkyles en C₁-C₆ et les radicaux hydroxyalkyles ou dihydroxyalkyles en C₁-C₆ ;
   - R₁₆ est choisi parmi :
      - le radical 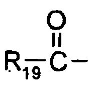
      - les radicaux R₂₀ hydrocarbonés en C₁-C₂₂ linéaires ou ramifiés, saturés ou insaturés,
      - l'atome d'hydrogène,
   - R₁₈ est choisi parmi :
      - le radical 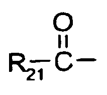
      - les radicaux R₂₂ hydrocarbonés en C₁-C₆ linéaires ou ramifiés, saturés ou insaturés,
      - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X⁻ est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (XV) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 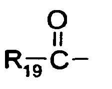
   - les radicaux méthyle, éthyle ou hydrocarbonés en C₁₄-C₂₂
   - l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi :
   - le radical 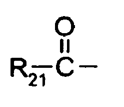
   - l'atome d'hydrogène ;

De tels composés sont par exemple commercialisés sous les dénominations DEHYQUART par la société COGNIS, STEPANQUAT par la société STEPAN, NOXAMIUM par la société CECA, REWOQUAT WE 18 par la société REWO-WITCO.

Parmi les sels d'ammonium quaternaire on préfère le chlorure de cétyltriméthylammonium, le chlorure de béhényl triméthylammonium ou encore, le chlorure palmitamidopropyl triméthyl ammonium commercialisé sous la dénomination VARISOFT PA TC par la société GOLDSCHMIDT.

On utilise de préférence comme agent tensioactif anionique les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélanges avec :
- soit un agent tensioactif amphotère tel que les dérivés d'amine dénommés disodiumcocoamphodiacetate ou sodiumcocoamphopropionate commercialisés notamment par la société RHODIA CHIMIE sous la dénomination commerciale "MIRANOL^{®} C2M CONCNP" en solution aqueuse à 38 % de matière active ou sous la dénomination MIRANOL^{®} C32;
- soit un agent tensioactif amphotère tel que les alkylbétaïnes en particulier la cocobétaïne commercialisée sous la dénomination "DEHYTON^{®} AB 30" en solution aqueuse à 32 % de MA par la société COGNIS ou tel que les alkyl (C₈-C₂₀) amidoalkyl (C₁-C₆) bétaïnes en particulier la TEGOBETAINE^{®} F 50 commercialisée par la société GOLDSCHMIDT.

Le milieu cosmétiquement acceptable peut être constitué uniquement par de l'eau ou par un mélange d'eau et d'un ou plusieurs solvants cosmétiquement acceptables ou par un ou plusieurs solvants cosmétiquement acceptables, tel qu'un alcool inférieur en C₁-C₄, comme l'éthanol, l'isopropanol, le tertiobutanol, le n-butanol; les alkylèneglycols comme le propylèneglycol, les éthers de glycols. L'eau représente de préférence de 30 à 98% en poids et de préférence de 50 à 98% en poids par rapport au poids total de la composition.

On peut citer plus particulièrement les monoalcools tels que l'éthanol, l'isopropanol, les polyalcools tels que le diéthylèneglycol, la glycérine, les éthers de glycol, les alkyléthers de glycol ou de diéthylèneglycol.

Les compositions selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants (associatifs ou non associatifs). On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir, de préférence, jusqu'à 5 % d'agents nacrants ou opacifiants bien connus dans l'état de la technique tels que par exemple les alcools gras supérieurs à C16, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions conformes à l'invention peuvent éventuellement contenir, en outre, au moins un additif choisi parmi les synergistes de mousses tels que des 1,2-alcanediols en C₁₀-C₁₈ ou des alcanolamides gras dérivés de mono ou de diéthanolamine, les filtres solaires siliconés ou non, les polymères anioniques, non ioniques, amphotères ou cationiques autres que les polyalkylèneimines de l'invention, les protéines, les hydrolysats de protéines, les hydroxyacides, les vitamines, les provitamines tels que le panthénol, les silicones volatiles ou non, linéaires ou cycliques, réticulées ou non, organomodifiées ou non, les huiles de synthèse telles que les poly-α-oléfines, les huiles fluorées, les cires fluorées, les gommes fluorées, les esters d'acides carboxyliques, les huiles minérales, végétales ou animales, les céramides, les pseudocéramides et leurs mélanges.

Bien entendu, l'homme du métier veillera à choisir ce ou ces éventuels composés complémentaires et/ou leurs quantités de manière telle que les propriétés avantageuses attachées intrinsèquement à l'association conforme à l'invention ne soient pas ou substantiellement pas, altérées par la ou les adjonctions envisagées.

Ces additifs sont, éventuellement, présents dans la composition selon l'invention dans des proportions pouvant aller de 0,00001 à 20% en poids par rapport au poids total de la composition. La quantité précise de chaque additif est déterminée facilement par l'homme du métier selon sa nature et sa fonction.

Les compositions peuvent se présenter sous forme de liquides fluides ou épaissis, de gels, de crèmes, de mousses, d'émulsions eau-dans l'huile (E/H), huile-dans-eau (H/E) ou d'émulsions multiples.

Elles peuvent être utilisées, par exemple, comme shampoings, soins rincés, masques de soin profond, lotions ou crèmes de traitement du cuir chevelu.

Ces compositions conviennent principalement au lavage, au soin des matières kératiniques en particulier des cheveux et de la peau et encore plus particulièrement des cheveux.
Les compositions de l'invention peuvent plus particulièrement se présenter sous forme de shampooing, d'après-shampooing à rincer ou non, de compositions pour permanente, défrisage, coloration ou décoloration, ou encore sous forme de compositions à appliquer avant ou après une coloration, une décoloration, une permanente ou un défrisage ou encore entre les deux étapes d'une permanente ou d'un défrisage. De préférence, les compositions sont des compositions lavantes et moussantes pour les cheveux et/ou la peau.

En particulier, les compositions selon l'invention sont des compositions détergentes moussantes telles que des shampooings, des gels-douche et des bains moussants. Dans ce mode de réalisation de l'invention, les compositions comprennent au moins un tensioactif détergent.

Le ou les tensioactifs détergents peuvent être indifféremment choisis, seuls ou en mélanges, au sein des tensioactifs anioniques, amphotères, non ioniques, zwittérioniques et cationiques décrits ci-dessus.

La quantité minimale tensioactif est celle juste suffisante pour conférer à la composition finale un pouvoir moussant et/ou détergent satisfaisant.
Ainsi, selon l'invention, le tensioactif détergent peut représenter de 3 % à 30 % en poids, de préférence de 6 % à 25 % en poids, et encore plus préférentiellement de 8 % à 20 % en poids, du poids total de la composition finale.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

L'invention a également pour objet un procédé de lavage et de conditionnement des matières kératiniques, telles que notamment les cheveux, consistant à appliquer une composition telle que définie ci-dessus, puis à effectuer un rinçage à l'eau après un éventuel temps de pause.

Les compositions selon l'invention sont utilisées, de préférence, comme shampooings pour le lavage et le conditionnement des cheveux et ils sont appliqués dans ce cas-là sur les cheveux humides dans des quantités efficaces pour les laver, cette application étant suivie d'un rinçage à l'eau.

Les compositions conformes à l'invention sont également utilisables comme gel- douche pour le lavage et le conditionnement des cheveux et/ou de la peau, auquel cas ils sont appliqués sur la peau et/ou les cheveux humides et sont rincés après application.

Les compositions de l'invention peuvent également être utilisées en non rincé, et en particulier dans des lotions, dans des gels, dans des mousses, dans des aérosols.

Selon l'invention, on prépare un pré-mélange des particules et de la polyalkylèneamine que l'on introduit dans la composition comprenant les tensioactifs.

Des exemples concrets illustrant l'invention vont maintenant être donnés.

### EXEMPLE 1:

On a réalisé la composition de shampooing ci-après :

| | |
|---|---|
| Lauryl sulfate de triéthanolamine en solution aqueuse à 40 % MA | 31,2 g |
| Alkyl (C1/C9) polyglucoside [1,4] en solution aqueuse à 40 %) | 6,25 g |
| Polyéthylèneimine (LUPASOL FG de BASF) Carbonate de calcium en poudre (OMYAPUR 35 | 0,015 g |
| commercialisé par OMYA) | 3 g |
| Agent de pH qsp | pH=7 |
| Eau qsp | 100 g |

La composition présente une texture agréable lors de l'application sur des cheveux humides. Sa rinçabilité est bonne. Les cheveux mouillés ne sont pas chargés et la mise en forme est aisée.

### EXEMPLE 2:

On a réalisé la composition de shampooing ci-après :

| | |
|---|---|
| Lauryl éthersulfate de sodium en solution aqueuse à 30% MA | 17,8 g |
| Cocoyl amidopropyl bétaïne en solution aqueuse à 30 % MA | 8,3g |
| Polyéthylèneimine (LUPASOL FG de BASF) | 0,015 g |
| Laponite en poudre LAPONITE XLGde LAPORTE) | 3 g |
| Agent de pH qsp | pH=7 |
| Eau qsp | 100 g |

La composition présente une texture agréable lors de l'application sur des cheveux humides. Sa rinçabilité est bonne. Les cheveux mouillés ne sont pas chargés et la mise en forme est aisée.

### EXEMPLE 3:

On a réalisé la lotion ci-après :

| | |
|---|---|
| Polyéthylèneimine (LUPASOL FG de BASF) | 0,015 g |
| Carbonate de calcium en poudre (OMYAPUR 35 commercialisé par OMYA) | 3 g |
| Agent de pH qsp | pH=7 |
| Eau qsp | 100 g |

On a mesuré les forces de frottement sur mèches de cheveux naturels séchés à l'aide d'un banc de glissement.

Une mèche mobile, fixée sur un banc de glissement, est entraînée dans un mouvement rectiligne horizontal entre deux autres mèches fixes. La force à exercer pour faire glisser la mèche est mesurée à l'aide d'une jauge électronique reliée au bras d'entraînement. Plus la force de glissement est importante, plus les effets du dépôt des particules est important. L'effet coiffant est renforcé.

Les résultats sont rassemblés dans le tableaux ci-dessous :

| | Exemple 3 avec carbonate de calcium | Comparatif (sans PEI) |
|---|---|---|
| Forces de frottement | 30,8 g | 25,2 g |

La force de glissement de la composition contenant le carbonate de calcium et la polyéthylèneimine (PEI) selon l'invention est nettement supérieure à celle de la composition sans PEI.

### EXEMPLE 4:

On a réalisé la lotion ci-après :

| | |
|---|---|
| Polyéthylèneimine (LUPASOL FG de BASF) | 0,015 g |
| Laponite (Silicate mixte sodium lithium magnésium) en poudre LAPONITE XLG de LAPORTE) | 3 g |
| Agent de pH qsp | pH=7 |
| Eau qsp | 100 g |

On a mesuré les forces de frottement sur mèches de cheveux naturels séchés à l'aide d'un banc de glissement.

Une mèche mobile, fixée sur un banc de glissement, est entraînée dans un mouvement rectiligne horizontal entre deux autres mèches fixes. La force à exercer pour faire glisser la mèche est mesurée à l'aide d'une jauge électronique reliée au bras d'entraînement. Plus la force de glissement est importante, plus les effets du dépôt des particules est important. L'effet coiffant est renforcé.

Les résultats sont rassemblés dans le tableaux ci-dessous :

| | Exemple 4 avec Laponite | Comparatif (sans PEI) |
|---|---|---|
| Forces de frottement | 25,7 g | 19,3 g |

La force de glissement de la composition contenant la laponite et la polyéthylèneimine (PEI) selon l'invention est nettement supérieure à celle de la composition sans PEI.

## Revendications

1. Composition cosmétique, notamment capillaire, comprenant, dans un milieu cosmétiquement acceptable :
- des particules minérales solides comprennent au moins un élément de la colonne IIa, du tableau de classification périodique des éléments et
- au moins une polyalkylèneimine,
le rapport pondéral polyalkylèneimine/ particules minérales allant de 0,1 à 0,0001.

2. Composition selon la revendication 1, **caractérisée par le fait que** les particules solides présentent une taille primaire moyenne en nombre comprise entre 2 nm et 2 microns, plus préférentiellement entre 5 et 500 nm, et plus préférentiellement encore entre 10 et 250 nm.

3. Composition selon l'une quelconque des revendications précédentes, **caractérisée par le fait que** les particules sont choisies parmi les particules contenant au moins 10 % en poids de carbonate de calcium ou d'au moins un silicate, l'oxyde de magnésium, le sulfate de baryum et leurs mélanges.

4. Composition selon la revendication 3, **caractérisée par le fait que** les particules contiennent au moins 50 % en poids de carbonate de calcium, mieux encore au moins 70 % en poids, et encore mieux plus de 90 % en poids de carbonate de calcium.

5. Composition selon la revendication précédente, **caractérisée par le fait que** les particules sont des particules de carbonate de calcium substantiellement pur.

6. Composition selon l'une quelconque des revendications précédentes, **caractérisée par le fait que** les particules solides sont utilisées en une quantité comprise entre 0,01 % et 30 % en poids, et de préférence entre 0,05 % et 10 % en poids par rapport au poids total de la composition.

7. Composition selon l'une quelconque des revendications précédentes, **caractérisée par le fait que** les polyalkylèneimines comprennent les motifs répétitifs suivants : 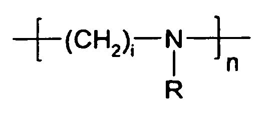 dans lesquels :
i représente un entier supérieur ou égal à 2 , préférentiellement i=2 ;
n représente un entier variant de 6 à 20.000 et de préférence de 8 à 2500,
R représente un atome d'hydrogène ou un motif 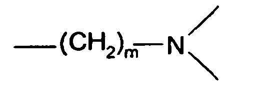
dans lequel m représente un entier supérieur ou égal à 2, préférentiellement m=2 ;

8. Composition selon l'une quelconque des revendications 1 à 7, **caractérisée par le fait que** la ou lesdites polyalkylèneimines sont des polyalkylène(C2-C4)imines et plus particulièrement des polyéthylèneimines.

9. Composition selon l'une quelconque des revendications 1 à 8, **caractérisée par le fait que** la ou lesdites polyalkylèneimines ont un poids moléculaire moyen en poids allant de 300 à 100.000 et de préférence de 350 à 50.000 et plus particulièrement de 400 à 10.000.

10. Composition selon l'une quelconque des revendications 1 à 9, **caractérisée par le fait que** la ou lesdites polyalkylèneimines sont présentes dans des proportions allant de 0,0005 à 5 % en poids et, de préférence, de 0,001 à 1 % en poids par rapport au poids total de la composition et plus particulièrement de 0,005 à 0,25% en poids.

11. Composition selon l'une quelconque des revendications précédentes, **caractérisée par le fait qu'**elle comprend, en outre, au moins un additif choisi parmi les synergistes de mousses tels que des 1,2-alcanediols en C₁₀-C₁₈ ou des alcanolamides gras dérivés de mono ou de diéthanolamine, les filtres solaires siliconés ou non, les agents tensioactifs autres que ceux de l'invention, les polymères anioniques, non ioniques, amphotères ou cationiques différents des polyalkylène imines, les protéines, les hydrolysats de protéines, les hydroxyacides, les vitamines, les provitamines tels que le panthénol, les silicones volatiles ou non, linéaires ou cycliques, réticulées ou non, organomodifiées ou non.

12. Utilisation d'une composition telle définie dans l'une quelconque des revendications 1 à 11 comme shampooing.

13. Utilisation d'une composition telle définie dans l'une quelconque des revendications 1 à 11 pour apporter de la texture aux cheveux (sensation d'épaisseur accrue) et une meilleure tenue de la coiffure.

14. Procédé de lavage et de conditionnement des cheveux consistant à appliquer une quantité efficace d'une composition telle que définie à l'une quelconque des revendications 1 à 11, puis à effectuer un rinçage à l'eau après un éventuel temps de pose.

## Claims

1. Cosmetic composition, especially a hair composition, comprising, in a cosmetically acceptable medium:
- solid mineral particles comprising at least one element from column IIa of the Periodic Table of the Elements, and
- at least one polyalkyleneimine,
the polyalkyleneimine/mineral particle weight ratio ranging from 0.1 to 0.0001.

2. Composition according to Claim 1, **characterized in that** the solid particles have a number-average primary size of between 2 nm and 2 microns, more preferably between 5 and 500 nm and even more preferably between 10 and 250 nm.

3. Composition according to either of the preceding claims, **characterized in that** the particles are chosen from particles containing at least 10% by weight of calcium carbonate or of at least one silicate, magnesium oxide, barium sulphate, and mixtures thereof.

4. Composition according to Claim 3, **characterized in that** the particles contain at least 50% by weight of calcium carbonate, better still at least 70% by weight and even better still more than 90% by weight of calcium carbonate.

5. Composition according to the preceding claim, **characterized in that** the particles are particles of substantially pure calcium carbonate.

6. Composition according to any one of the preceding claims, **characterized in that** the solid particles are used in an amount of between 0.01% and 30% by weight and preferably between 0.05% and 10% by weight relative to the total weight of the composition.

7. Composition according to any one of the preceding claims, **characterized in that** the polyalkyleneimines comprise the following repeating units: 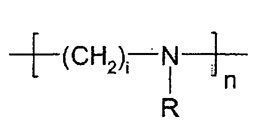 in which:
i represents an integer greater than or equal to 2,
preferentially, i=2;
n represents an integer ranging from 6 to 20 000 and preferably from 8 to 2 500;
R represents a hydrogen atom or a unit 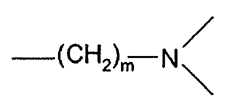
in which m represents an integer greater than or equal to 2; preferentially, m=2.

8. Composition according to any one of Claims 1 to 7, **characterized in that** the said polyalkyleneimine(s) is (are) poly(C2-C4)alkyleneimines and more particularly polyethyleneimines.

9. Composition according to any one of Claims 1 to 8, **characterized in that** the said polyalkyleneimine(s) has (have) a weight-average molecular weight ranging from 300 to 100 000, preferably from 350 to 50 000 and more particularly from 400 to 10 000.

10. Composition according to any one of Claims 1 to 9, **characterized in that** the said polyalkyleneimine(s) is (are) present in proportions ranging from 0.0005% to 5% by weight, preferably from 0.001% to 1% by weight and more particularly from 0.005% to 0.25% by weight relative to the total weight of the composition.

11. Composition according to any one of the preceding claims, **characterized in that** it also comprises at least one additive chosen from foam synergists such as C₁₀-C₁₈ 1,2-alkanediols or fatty alkanolamides derived from monoethanolamine or diethanolamine, silicone-based or non-silicone-based sunscreens, surfactants other than those of the invention, anionic, nonionic, amphoteric or cationic polymers other than the polyalkyleneimines, proteins, protein hydrolysates, hydroxy acids, vitamins, provitamins such as panthenol, volatile or nonvolatile, linear or cyclic, crosslinked or non-crosslinked, organomodified or non-organomodified silicones.

12. Use of a composition as defined in any one of Claims 1 to 11, as a shampoo.

13. Use of a composition as defined in any one of Claims 1 to 11, to give the hair texture (greater sensation of thickness) and better hold of the hairstyle.

14. Process for washing and conditioning the hair, which consists in applying an effective amount of a composition as defined in any one of Claims 1 to 11 and then in rinsing with water, after an optional action time.

## Patentansprüche

1. Kosmetische Zusammensetzung, insbesondere für die Haarbehandlung, die in einem kosmetisch akzeptablen Medium enthält:
- feste anorganische Partikel, die mindestens ein Element der Gruppe IIa des Periodensystems der Elemente enthalten, und
- mindestens ein Polyalkylenimin,
wobei das Gewichtsverhältnis Polyalkylenimin/anorganische Partikel im Bereich von 0,1 bis 0,0001 liegt.

2. Zusammensetzung nach Anspruch 1, **dadurch gekennzeichnet, dass** die festen Partikel eine zahlenmittlere Primärgröße von 2 nm bis 2 µm, vorzugsweise 5 bis 500 nm und noch bevorzugter 10 bis 250 nm aufweisen.

3. Zusammensetzung nach einem der vorhergehenden Ansprüche, **dadurch gekennzeichnet, dass** die Partikel unter den Partikeln ausgewählt sind, die mindestens 10 Gew.-% Calciumcarbonat oder mindestens ein Silicat, Magnesiumoxid, Bariumsulfat und deren Gemische enthalten.

4. Zusammensetzung nach Anspruch 3, **dadurch gekennzeichnet, dass** die Partikel mindestens 50 Gew.-% Calciumcarbonat, besser mindestens 70 Gew.-% und noch besser mehr als 90 Gew.-% Calciumcarbonat enthalten.

5. Zusammensetzung nach dem vorhergehenden Anspruch, **dadurch gekennzeichnet, dass** die Partikel im Wesentlichen reine Calciumcarbonatpartikel sind.

6. Zusammensetzung nach einem der vorhergehenden Ansprüche, **dadurch gekennzeichnet, dass** die festen Partikel in einer Menge von 0,01 bis 30 Gew.-% und vorzugsweise 0,05 bis 10 Gew.-%, bezogen auf das Gesamtgewicht der Zusammensetzung, verwendet werden.

7. Zusammensetzung nach einem der vorhergehenden Ansprüche, **dadurch gekennzeichnet, dass** die Polyalkylenimine die folgenden Wiederholungseinheiten enthalten: 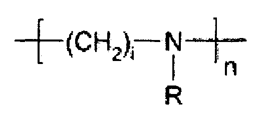 worin bedeuten:
i 2 oder eine ganze Zahl über 2, vorzugsweise i=2;
n eine ganze Zahl von 6 bis 20.000 und vorzugsweise 8 bis 2.500,
R ein Wasserstoffatom oder eine Einheit 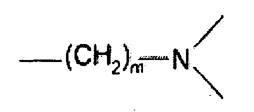
wobei m 2 oder eine ganze Zahl über 2 bedeutet, vorzugsweise m=2.

8. Zusammensetzung nach einem der Ansprüche 1 bis 7, **dadurch gekennzeichnet, dass** das Polyalkylenimin oder die Polyalkylenimine Polyalkylen(C₂₋₄)imine und insbesondere Polyethylenimine sind.

9. Zusammensetzung nach einem der Ansprüche 1 bis 8, **dadurch gekennzeichnet, dass** das oder die Polyalkylenimin(e) eine gewichtsmittlere Molmasse von 300 bis 100.000, vorzugsweise 350 bis 50.000 und insbesondere 400 bis 10.000 aufweisen.

10. Zusammensetzung nach einem der Ansprüche 1 bis 9, **dadurch gekennzeichnet, dass** das oder die Polyalkylenimin(e) in Mengenanteilen von 0,0005 bis 5 Gew.-% und vorzugsweise 0,001 bis 1 Gew.-%, bezogen auf das Gesamtgewicht der Zusammensetzung, und insbesondere 0,005 bis 0,25 Gew.-% enthalten sind.

11. Zusammensetzung nach einem der vorhergehenden Ansprüche, **dadurch gekennzeichnet, dass** sie ferner mindestens einen Zusatzstoff enthält, der unter den Schaumsynergisten, wie 1,2-Alkandiolen mit 10 bis 18 Kohlenstoffatomen oder von Mono- oder Diethanolamin abgeleiteten Alkanolfettamiden, siliconierten oder nicht siliconierten Sonnenschutzfiltern, von den erfindungsgemäßen Verbindungen verschiedenen grenzflächenaktiven Stoffen, anionischen, nichtionischen, amphoteren oder kationischen Polymeren, die von den Polyalkyleniminen verschieden sind, Proteinen, Proteinhydrolysaten, Hydroxysäuren, Vitaminen, Provitaminen, wie Panthenol, flüchtigen oder nicht flüchtigen, linearen oder cyclischen, vernetzten oder nicht vernetzten, organomodifizierten oder nicht organomodifizierten Siliconen ausgewählt ist.

12. Verwendung einer Zusammensetzung nach einem der Ansprüche 1 bis 11 als Haarwaschmittel.

13. Verwendung einer Zusammensetzung nach einem der Ansprüche 1 bis 11, um dem Haar Textur (Gefühl der größeren Dicke) und der Frisur einen besseren Halt zu geben.

14. Verfahren zur Reinigung und Konditionierung der Haare, das darin besteht, eine wirksame Menge einer Zusammensetzung nach einem der Ansprüche 1 bis 11 aufzutragen und anschließend nach einer gegebenenfalls abgewarteten Einwirkzeit mit Wasser zu spülen.
